# EUROPEAN PATENT APPLICATION

(11) **EP 1 238 644 A1**
(43) Date of publication of application: **11.09.2002**
(21) Application number: 00981715.6
(22) Date of filing: 14.12.2000
(51) Int. Cl.: A61K 7/00, A61K 31/375, A61K 31/665, A61K 47/02, A61K 47/18, A61K 47/20

(54) **STABILIZERS FOR L-ASCORBIC ACID-2-SODIUM PHOSPHATE**

(30) Priority: 15.12.1999 JP 35536799
(71) Applicant: KYOWA HAKKO KOGYO CO., LTD., Chiyoda-ku, Tokyo 100-8185 (JP)
(72) Inventor: NAGAMURA, Satoru, c/o Technical Research Lab., Hofu-shi, Yamaguchi 747-8522 (JP); MATSUDA, Hisashi, c/o Technical Research Lab., Hofu-shi, Yamaguchi 747-8522 (JP)
(74) Representative: Casalonga, Axel
(86) International application number: JP0008853
(87) International publication number: WO01043702

(57) **Abstract**

The present invention provides a stabilizer for sodium L-ascorbate-2-phosphate comprising:
(1) a compound having a thiol group or a disulfide bond,
(2) sulfurous acid or its salt, or
(3) an amino acid having a hydroxyl group.

## Description

### Technical Field

The present invention relates to a stabilizer for sodium L-ascorbate-2-phosphate.

### Background Art

Ascorbic acid, which is known as an antiscorbutic factor, has been reported to have whitening activity by inhibiting melanin pigmentation which causes skin stains, freckles, etc., to prevent wrinkles by promoting collagen metabolism, to moisturize skin, and recently, to have a carcinostatic effect.

However, ascorbic acid and its derivatives, which are relatively stable in weakly acidic aqueous solutions, have the properties of decomposing easily, losing their function, and coloring in neutral or alkaline aqueous solutions.

Japanese Published Unexamined Patent Application No. 118613/97 discloses cosmetics comprising sodium L-ascorbate-2-phosphate and a divalent or higher metal salt which were developed with the aim of stabilizing ascorbic acid and its derivatives.

Also, Japanese Published Unexamined Patent Application No. 82127/95 discloses cosmetics comprising an L-ascorbic acid derivative and a basic amino acid such as lysine or arginine.

Japanese Published Unexamined Patent Application No. 1414/98 discloses skin cosmetics comprising an ascorbic acid derivative and serine etc., but there has been no report on the stabilizing activity of serine etc., on an ascorbic acid derivative.

### Disclosure of the Invention

An object of the present invention is to provide a stabilizer for sodium L-ascorbate-2-phosphate.

The present invention relates to the following 1 to 11.
1. A stabilizer for sodium L-ascorbate-2-phosphate comprising:
   (1) a compound having a thiol group or a disulfide bond,
   (2) sulfurous acid or its salt, or
   (3) an amino acid having a hydroxyl group.
2. A stabilizer for sodium L-ascorbate-2-phosphate comprising a compound having a thiol group or a disulfide bond.
3. The stabilizer for sodium L-ascorbate-2-phosphate according to the above 1 or 2, wherein the compound having a thiol group or a disulfide bond is reduced glutathione, cysteine or methionine.
4. A stabilizer for sodium L-ascorbate-2-phosphate comprising sulfurous acid or its salt.
5. The stabilizer for sodium L-ascorbate-2-phosphate according to the above 1 or 4, wherein the sulfurous acid or its salt is sodium sulfite or sodium hydrogen sulfite.
6. A stabilizer for sodium L-ascorbate-2-phosphate comprising an amino acid having a hydroxyl group.
7. The stabilizer for sodium L-ascorbate-2-phosphate according to the above 1 or 6, wherein the amino acid having a hydroxyl group is serine or threonine.
8. A method for stabilizing sodium L-ascorbate-2-phosphate which comprises adding thereto:
   (1) a compound having a thiol group or a disulfide bond,
   (2) sulfurous acid or its salt, or
   (3) an amino acid having a hydroxyl group.
9. A composition comprising sodium L-ascorbate-2-phosphate, and
   (1) a compound having a thiol group or a disulfide bond, or
   (2) sulfurous acid or its salt.
10. A cosmetic comprising sodium L-ascorbate-2-phosphate, and
   (1) a compound having a thiol group or a disulfide bond, or
   (2) sulfurous acid or its salt.
11. A composition for the oral cavity comprising sodium L-ascorbate-2-phosphate, and
   (1) a compound having a thiol group or a disulfide bond,
   (2) sulfurous acid or its salt, or
   (3) an amino acid having a hydroxyl group.

In the present invention, there is no specific restriction as to the compound having a thiol group or a disulfide bond so long as it has a thiol group or a disulfide bond in the molecule, but water-soluble ones are preferred. Suitable examples are reduced glutathione, cysteine, dithiothreitol, mercaptoethanol, ethanedithiol and methionine. Preferred are reduced glutathione, cysteine and methionine.

The compound having a thiol group or a disulfide bond is used preferably in an amount of 0.001 to 10 molar equivalents, more preferably 0.1 to 0.5 molar equivalent based on sodium L-ascorbate-2-phosphate.

In the present invention, the sulfites include salts of sulfurous acid with an alkali metal or an alkaline earth metal, preferably salts with an alkali metal, more preferably salts with sodium or potassium. Suitable examples are sodium sulfite, sodium hydrogen sulfite, potassium sulfite and potassium hydrogen sulfite. Preferred are sodium sulfite and sodium hydrogen sulfite.

Sulfurous acid or its salt is used preferably in an amount of 0.001 to 10 molar equivalents, more preferably 0.01 to 1 molar equivalent, further preferably 0.1 to 0.5 molar equivalent based on sodium L-ascorbate-2-phosphate.

In the present invention, the amino acids having a hydroxyl group include, for example, serine, threonine and tyrosine. Preferred are serine and threonine.

The amino acid having a hydroxyl group is used preferably in an amount of 0.001 to 10 molar equivalents, more preferably 0.01 to 1 molar equivalent, futher preferably 0.1 to 0.5 molar equivalent based on sodium L-ascorbate-2-phosphate.

When using the stabilizer of the present invention, it is added, for example, to an aqueous solution of sodium L-ascorbate-2-phosphate. It is effective when used in aqueous solutions of pH 3 to 9, especially pH 3 to 7.

The cosmetic of the present invention can be prepared, for example, in a method similar to those described in Japanese Published Unexamined Patent Application No. 82127/95, Japanese Published Unexamined Patent Application No. 118613/97, Japanese Published Unexamined Patent Application No. 1414/98, etc., with addition of a pigment, a perfume, an antiseptic, a surfactant, an antioxidant, an ultraviolet absorbent or the like, according to need.

Examples of the pigments are tar pigments, iron oxide, titanium oxide and zinc oxide.

Examples of the perfumes are animal perfumes such as musk, vegetable perfumes such as peppermint oil, lemon oil and rose oil, and synthetic perfumes such as benzyl alcohol and anisole.

Examples of the antiseptics are paraben, methylparaben, ethyl p-oxybenzoate and butyl p-oxybenzoate.

Examples of the surfactants are anionic surfactants such as sodium cetylsulfate, nonionic surfactants such as polyoxyethylene alkyl ethers, polyoxyethylene fatty acid esters, polyoxyethylene polyhydric alcohol fatty acid esters, fatty acid ester hardened castor oil, polyhydric alcohol fatty acid esters and polyglycerin fatty acid esters, cationic surfactants such as tetraalkylammonium salts, and amphoteric surfactants such as betaine surfactants, sulfobetaine surfactants, sulfoamino acid surfactants and sodium N-stearoyl-L-glutamate.

An example of the antioxidant is dibutylhydroxytoluene.

Examples of the ultraviolet absorbents are 2-ethylhexyl para-methoxycinnamate and 4-tert-butyl-4'-methoxydibenzoylmethane.

The cosmetic of the present invention can be prepared in the form of cream, emulsion, lotion, enriched lotion, pack, lotion, powder, or the like. In the case of emulsion, for example, the cosmetic can be produced according to a known method wherein the oil phase and the water phase respectively heated are emulsified, followed by cooling.

The content of sodium L-ascorbate-2-phosphate in the cosmetic of the present invention is preferably 0.001 to 10 wt%, more preferably 0.01 to 5 wt%.

The above-described pigments, perfumes, antiseptics, surfactants, antioxidants, ultraviolet absorbents, etc. are added in such range of quantity that the object of the present invention can be achieved.

The composition for the oral cavity of the present invention can be prepared, for example, in a method similar to that described in Japanese Published Unexamined Patent Application No. 99849/96 etc., with addition of a surfactant, a thickener, a viscous agent, a sweetener, a flavor, an antiseptic, a polishing material, a wetting agent or the like, if necessary.

Examples of the surfactants are anionic surfactants such as sodium alkylsulfates, sodium N-acylsarcosinates and N-acylglutamic acids, and nonionic surfactants such as sugar fatty acid esters, sugar alcohol fatty acid esters, polyoxyethylene fatty acid esters and fatty acid ethanolamides.

Examples of the thickeners are cellulose derivatives such as sodium carboxycellulose and methyl cellulose, gums such as xanthane gum, tragacanth gum, karaya gum and gum arabic, and synthetic thickeners such as polyvinyl pyrrolidone.

Examples of the viscous agents are sorbitol, glycerin, ethylene glycol and xylitol.

Examples of the sweeteners are aspartylphenylalanine methyl ester and stevioside.

Examples of the flavors are saccharin sodium, peppermint oil, spearmint oil and menthol.

Examples of the antiseptics are ethyl p-oxybenzoate and butyl p-oxybenzoate.

Examples of the polishing materials are calcium carbonate, calcium hydrogenphosphate, silicic acid anhydride and aluminium hydroxide.

Examples of the wetting agents are glycerin and sorbitol.

The composition for the oral cavity of the present invention can be prepared in the form of dentifrice, mouthwash, gingival massage cream, liquid or paste for local application, chewing gum, or the like.

The content of sodium L-ascorbate-2-phosphate in the composition for the oral cavity of the present invention is preferably 0.001 to 10 wt%, more preferably 0.01 to 5 wt%.

The above-described surfactants, thickeners, viscous agents, sweeteners, flavors, antiseptics, polishing materials, wetting agents, etc. are added in such range of quantity that the object of the present invention can be achieved.

In the practice of the method for stabilization and the composition of the present invention, conditions similar to those for the stabilizer etc. described above can be applied.

Certain embodiments of the present invention are illustrated in the following examples.

### Modes for Carrying Out the Invention

### Example 1

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid and a 0.1 mol/l aqueous solution of sodium hydroxide. To this solution was added water to prepare a 30 g/l solution of sodium L-ascorbate-2-phosphate (0.084 mol/l), followed by addition of 0.1 mol of reduced glutathione. The resulting solution was allowed to stand at 60°C, and after 0, 3 and 6 days, the absorbance (OD) was measured at 430 nm (test sample). A solution prepared in the same manner as above except that reduced glutathione was not added was used as a control. The results are shown in Table 1.

**Table 1**

| | Control | Test sample |
|---|---|---|
| Day 0 | 0.0031 | 0.0031 |
| Day 3 | 1.0379 | 0.0068 |
| Day 6 | 1.4928 | 0.0273 |

### Example 2

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid and a 0.1 mol/l aqueous solution of sodium hydroxide. To this solution was added water to prepare a 30 g/l solution of sodium L-ascorbate-2-phosphate (0.084 mol/l), followed by addition of cysteine in an amount of 0.1 mol (test sample-1) or 0.01 mol (test sample-2). Each of the resulting solutions was allowed to stand at 60°C, and after 0, 3 and 6 days, the OD was measured at 430 nm. A solution prepared in the same manner as above except that cysteine was not added was used as a control. The results are shown in Table 2.

**Table 2**

| | Control | Test sample-1 | Test sample-2 |
|---|---|---|---|
| Day 0 | 0.0031 | 0.0031 | 0.0031 |
| Day 3 | 1.0379 | 0.0074 | 0.0354 |
| Day 6 | 1.4928 | 0.0363 | 0.5618 |

### Example 3

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid and a 0.1 mol/l aqueous solution of sodium hydroxide. To this solution was added water to prepare a 30 g/l solution of sodium L-ascorbate-2-phosphate (0.084 mol/l), followed by addition of sodium sulfite in an amount of 0.1 mol (test sample-1) or 0.02 mol (test sample-2). Each of the resulting solutions was allowed to stand at 60°C, and after 0, 3 and 6 days, the OD was measured at 430 nm. A solution prepared in the same manner as above except that sodium sulfite was not added was used as a control. The results are shown in Table 3.

**Table 3**

| | Control | Test sample-1 | Test sample-2 |
|---|---|---|---|
| Day 0 | 0.0031 | 0.0031 | 0.0031 |
| Day 3 | 1.0379 | 0.0023 | 0.0043 |
| Day 6 | 1.4928 | 0.0043 | 0.0995 |

### Example 4

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid and a 0.1 mol/l aqueous solution of sodium hydroxide. To this solution was added water to prepare a 30 g/l solution of sodium L-ascorbate-2-phosphate (0.084 mol/l), followed by addition of sodium hydrogen sulfite in an amount of 0.1 mol (test sample-1) or 0.02 mol (test sample-2). Each of the resulting solutions was allowed to stand at 60°C, and after 0, 3 and 6 days, the OD was measured at 430 nm. A solution prepared in the same manner as above except that sodium hydrogen sulfite was not added was used as a control. The results are shown in Table 4.

**Table 4**

| | Control | Test sample-1 | Test sample-2 |
|---|---|---|---|
| Day 0 | 0.0031 | 0.0031 | 0.0031 |
| Day 3 | 1.0379 | 0.0026 | 0.0037 |
| Day 6 | 1.4928 | 0.0042 | 0.0779 |

### Example 5

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid. To this solution was added water to prepare a 30 g/l solution of sodium L-ascorbate-2-phosphate (0.084 mol/l), followed by addition of 0.1 mol of serine. The resulting solution was allowed to stand at 60°C, and after 0 and 3 days, the OD was measured at 430 nm. A solution prepared in the same manner as above except that serine was not added was used as a control. The results are shown in Table 5.

**Table 5**

| | Control | Test sample |
|---|---|---|
| Day 0 | 0.0039 | 0.0039 |
| Day 3 | 1.0344 | 0.5187 |

### Example 6

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid. To this solution was added water to prepare a 30 g/l solution of sodium L-ascorbate-2-phosphate (0.084 mol/l), followed by addition of 0.1 mol of threonine. The resulting solution was allowed to stand at 60°C, and after 0 and 3 days, the OD was measured at 430 nm. A solution prepared in the same manner as above except that threonine was not added was used as a control. The results are shown in Table 6.

**Table 6**

| | Control | Test sample |
|---|---|---|
| Day 0 | 0.0039 | 0.0039 |
| Day 3 | 1.0344 | 0.4827 |

### Example 7

In water were dissolved 1.5 g of sodium L-ascorbate-2-phosphate and 0.05 g of methylparaben as an antiseptic, and the resulting solution was adjusted to pH 5 with a 0.1 mol/l aqueous solution of hydrochloric acid. To this solution was added water to prepare a 30 g/l solution of sodium L-ascorbate-2-phosphate (0.084 mol/l), followed by addition of 0.1 mol of methionine. The resulting solution was allowed to stand at 60°C, and after 0 and 3 days, the OD was measured at 430 nm. A solution prepared in the same manner as above except that methionine was not added was used as a control. The results are shown in Table 7.

**Table 7**

| | Control | Test sample |
|---|---|---|
| Day 0 | 0.0031 | 0.0031 |
| Day 3 | 1.0379 | 0.3460 |

### Example 8

In water were dissolved 1.5 g of sodium L-ascorbate-2-phosphate and 0.05 g of methylparaben as an antiseptic, and the resulting solution was adjusted to pH 6 with a 0.1 mol/l aqueous solution of hydrochloric acid and an aqueous solution of sodium hydroxide. To this solution was added water to prepare a 30 g/l solution of sodium L-ascorbate-2-phosphate (0.084 mol/l), followed by addition of 0.1 mol of sodium sulfite (test sample-1) or 0.1 mol of sodium hydrogen sulfite (test sample-2). Each of the resulting solutions was allowed to stand at 60°C, and after 0 and 3 days, the OD was measured at 430 nm. A solution prepared in the same manner as above except that sodium sulfite or sodium hydrogen sulfite was not added was used as a control. The results are shown in Table 8.

**Table 8**

| | Control | Test sample-1 | Test sample-2 |
|---|---|---|---|
| Day 0 | 0.0031 | 0.0031 | 0.0031 |
| Day 3 | 0.8106 | 0.0070 | 0.0070 |

### Example 9

In water were dissolved 1.5 g of sodium L-ascorbate-2-phosphate and 0.05 g of methylparaben as an antiseptic, and the resulting solution was adjusted to pH 7 with a 0.1 mol/l aqueous solution of hydrochloric acid and a 0.1 mol/l aqueous solution of sodium hydroxide. To this solution was added water to prepare a 30 g/l solution (0.084 mol/l), followed by addition of 0.1 mol of sodium sulfite (test sample-1) or 0.1 mol of sodium hydrogen sulfite (test sample-2). Each of the resulting solutions was allowed to stand at 60°C, and after 0 and 3 days, the OD was measured at 430 nm. A solution prepared in the same manner as above except that sodium sulfite or sodium hydrogen sulfite was not added was used as a control. The results are shown in Table 9.

**Table 9**

| | Control | Test sample-1 | Test sample-2 |
|---|---|---|---|
| Day 0 | 0.0031 | 0.0031 | 0.0031 |
| Day 3 | 0.0905 | 0.0170 | 0.0167 |

The results of the above examples indicate that the stabilizer for sodium L-ascorbate-2-phosphate of the present invention prevents decomposition of sodium L-ascorbate-2-phosphate and inhibits coloring of a solution containing sodium L-ascorbate-2-phosphate.

### Industrial Applicability

The present invention provides a stabilizer for sodium L-ascorbate-2-phosphate.

## Claims

1. A stabilizer for sodium L-ascorbate-2-phosphate comprising:
(1) a compound having a thiol group or a disulfide bond,
(2) sulfurous acid or its salt, or
(3) an amino acid having a hydroxyl group.

2. A stabilizer for sodium L-ascorbate-2-phosphate comprising a compound having a thiol group or a disulfide bond.

3. The stabilizer for sodium L-ascorbate-2--phosphate according to Claim 1 or 2, wherein the compound having a thiol group or a disulfide bond is reduced glutathione, cysteine or methionine.

4. A stabilizer for sodium L-ascorbate-2-phosphate comprising sulfurous acid or its salt.

5. The stabilizer for sodium L-ascorbate-2-phosphate according to Claim 1 or 4, wherein the sulfurous acid or its salt is sodium sulfite or sodium hydrogen sulfite.

6. A stabilizer for sodium L-ascorbate-2-phosphate comprising an amino acid having a hydroxyl group.

7. The stabilizer for sodium L-ascorbate-2-phosphate according to Claim 1 or 6, wherein the amino acid having a hydroxyl group is serine or threonine.

8. A method for stabilizing sodium L-ascorbate-2-phosphate which comprises adding thereto:
(1) a compound having a thiol group or a disulfide bond,
(2) sulfurous acid or its salt, or
(3) an amino acid having a hydroxyl group.

9. A composition comprising sodium L-ascorbate-2-phosphate, and
(1) a compound having a thiol group or a disulfide bond, or
(2) sulfurous acid or its salt.

10. A cosmetic comprising sodium L-ascorbate-2-phosphate, and
(1) a compound having a thiol group or a disulfide bond, or
(2) sulfurous acid or its salt.

11. A composition for the oral cavity comprising sodium L-ascorbate-2-phosphate, and
(1) a compound having a thiol group or a disulfide bond,
(2) sulfurous acid or its salt, or
(3) an amino acid having a hydroxyl group.
